# EUROPÄISCHE PATENTSCHRIFT

(11) **EP 0 880 951 B1**
(45) Veröffentlichungstag und Bekanntmachung des Hinweises auf die Patenterteilung: **03.12.2003**
(21) Anmeldenummer: 98102651.1
(22) Anmeldetag: 16.02.1998
(51) Int. Cl.: A61F 2/52, B29C 35/02

(54) **Verfahren zur Herstellung einer Prothese für die weibliche Brust**
Method of producing a prosthesis for the female breast
Procédé de fabrication d'une prothèse mammaire pour femmes

(30) Priorität: 03.05.1997 DE 19718851; 05.12.1997 DE 19754144
(43) Veröffentlichungstag der Anmeldung: 02.12.1998
(73) Patentinhaber: Thämert Orthopädische Hilfsmittel GmbH & Co., 30938 Burgwedel (DE)
(72) Erfinder: Schneider-Nieskens, Reinhold, 21365 Adendorf (DE)
(74) Vertreter: Jabbusch, Wolfgang, Dr.Jur. Jabbusch, Wehser & Lauerwald Patentanwälte

(56) Entgegenhaltungen:
- EP-A- 0 433 636
- EP-A- 0 768 068
- US-A- 4 195 639
- US-A- 4 950 291

## Beschreibung

Die Erfindung betrifft ein Verfahren zur Herstellung einer Brustprothese mit einem etwa schalenförmigen Prothesenkörper, der gebildet wird durch Aufeinanderlegen von wenigstens zwei Kunststoffolien, die durch Randverbindung zu einem beutelartigen Behältnis mit mindestens einer Kammer ausgebildet werden und jede Kammer mit weichelastischem Material gefüllt wird, das nach Einlegen in ein Formwerkzeug einer Vulkanisierung unterzogen wird. Ein Verfahren gemäß dem Oberbegriff des Anspruchs 1 ist z.B. aus US-A-4 950 291 bekannt.

Prothesen für die weibliche Brust werden verwendet, um optisch wahrnehmbare Folgen chirurgischer Eingriffe auszugleichen bzw. zu verdecken. Üblicherweise bestehen die schalenförmigen Prothesenkörper von Brustprothesen aus einem weichelastischem Werkstoff, hauptsächlich Silikon-Kautschuk. Ein solcher Werkstoff weist relativ hohe Dichte auf, was sich nachteilig bei großen Größen (Gewicht) auf den Tragekomfort der Prothese auswirkt. Um das Gewicht einer Brustprothese zu vermindern, ist es bekannt, die verwendeten Silikon-Kautschuk-Massen mit Leichtfüllstoffen zu vermischen und aus der Mischung, die Prothesenkörper zu formen.

Die Leichtfüllstoffe verändern die Eigenschaften des Silikons, was sich bei einer Brustprothese nachteilig auswirkt. So ist zum Beispiel durch die innere Reibung der ausvulkanisierten Masse die Hysterese erheblich erhöht. Außerdem haftet ein mit Leichtfüllstoffen versetztes Silikon wesentlich schlechter an umgebenden Schutzhäuten, zum Beispiel Folien, die auch die Aufgabe miterfüllen sollen, den Prothesenkörper in Form zu halten, jedoch diese Aufgabe nicht erfüllen können, wenn die Leichtfüllstoffe in der Silikon-Kautschuk-Masse als die Haftung an Folien verschlechterndes Trennmittel wirken.

Des weiteren gestaltet sich die Farbgebung einer mit Füllstoffen versetzten Masse schwierig, weil die Füllstoffe durch Totalreflexion wie Weißpigmente wirken. Die für die Brustprothesen verwendete Masse kann deshalb nicht, in an und für sich zweckmäßiger Weise lasierend, sondern sie muß deckend eingefärbt werden, was zu einem optisch ungünstigen Erscheinungsbild der Brustprothese führt.

Der Erfindung liegt die Aufgabe zugrunde, ein Verfahren zu finden, mit dem in einfacher Weise eine aus einem schalenförmigen Prothesenkörper bestehende "Leichtprothese" mit verbesserten Eigenschaften herstellbar ist.

Diese Aufgabe ist erfindungsgemäß dadurch gelöst, daß zunächst wenigstens eine Außenkammer mit einem ersten Material gefüllt wird, daß das in wenigstens einer der Außenkammern befindliche Material außerhalb des Formwerkzeugs zumindest teilweise ausvulkanisiert wird, daß anschließend wenigstens eine Innenkammer mit einem zweiten Material gefüllt wird und daß danach der so vorbereitete Prothesenkörper im Formwerkzeug ausvulkanisiert wird.

Der Innenkern aus einem zweiten Material hat den Hauptanteil an dem Gewicht des Prothesenkörpers bzw. am Gesamtgewicht der Brustprothese. Durch die erfindungsgemäß mittels der Außenkammern gebildete Abdeckschicht aus einem ersten Material erhält der Prothesenkörper in seiner Ausgestaltung eine Annäherung an natürliches Gewebe, indem die Abdeckschicht eine Stütz- und Schutzfunktion für den Innenkern übernimmt, die der natürlichen menschlichen Haut in etwa entspricht und sich in etwa wie natürliche menschliche Haut auch anfühlt. Die Hysterese der Brustprothese und die mechanische Festigkeit ist verbessert. Auch die Farbgebung ist problemloser, da das erste Material für die Außenkammern lasierend, und damit ohne weiteres natürlich wirkend, eingefärbt werden kann.

Das zweite Material, aus dem ein von der Innenkammer gebildeter Innenkern besteht, ist eine mit 10 bis 25 %, vorzugsweise 18 %, Leichtfüllstoffen versetzte Silikon-Masse. Der Innenkern hat eine Dichte von 0,3 g/cm³ bis 0,8 g/cm³, vorzugsweise 0,64 g/cm³. Damit weist der Innenkern eine nicht unwesentlich geringere Dichte als die durchschnittliche Dichte einer natürlichen weiblichen Brust auf, die etwa bei 0,9 bis 1 g/cm³ liegt. Die Brustprothese ist folglich leicht und bietet ein angenehmes Tragegefühl, weil die Träger eines Büstenhalters, der die Prothese aufnimmt, entlastet werden. Dies kommt vor allem Frauen, die eine größere Brustprothese benötigen, zugute.

Als Leichtfüllstoffe werden bevorzugt Mikro-Glaskugeln eingesetzt mit einer Teilchengröße von 20 bis 100 µ, vorzugsweise etwa 75 µ. Selbstverständlich sind auch organische Füllstoffe einsetzbar.

Der Innenkern kann auch aus einem Polyurethanschaum bestehen oder aus einem Silikon-Schaum. Auch dadurch sind Reduzierungen des Gewichtes einer Brustprothese erreichbar.

Bei der erfindungsgemäßen Herstellung einer Brustprothese mit einem Innenkern, der mit wenigstens einer Abdeckschicht versehen wird, sind jedoch Gewichtsreduktionen von 20 - 40 % erreichbar, ohne das gewohnte Erscheinungsbild einer Brustprothese nachteilig zu beeinflussen.

Das erste Material, aus dem die mittels der Außenkammern gebildeten Abdeckschichten bestehen, ist eine übliche additionsvernetzte Zwei-Komponenten-Silikon-Kautschuk-Masse. Diese kann in gewohnter Weise verarbeitet und angewendet werden.

Der mittels einer Innenkammer gebildete Innenkern kann sowohl an seiner konvex als auch an seiner konkav gewölbten Oberfläche mit einer Abdeckschicht versehen werden. Da sich die für die Abdeckschicht verwendeten Silikon-Kautschuk-Massen ohne weiteres hinsichtlich ihrer Elastizität unterschiedlich einstellen lassen, kann zum Beispiel die Abdeckschicht an der konvexen Oberfläche etwas härter eingestellt sein, um die stützende Funktion einer natürlichen Haut besser nachahmen zu können.

Die an der konkav gewölbten Oberfläche vorhandene Abdeckschicht kann dagegen mit Vorteil besonders weich eingestellt werden, was der Verbesserung des Tragekomforts dienlich ist. Dabei kann, was wiederum für die Gewichtsreduzierung vorteilhaft ist, die konkave Außenschicht, etwa 20 - 70 %, vorzugsweise 50 % weniger Menge des ersten Materials, bezogen auf die Menge des ersten Materials für die konvexe Abdeckschicht, enthalten. Dadurch entstehen unterschiedlich dicke Abdeckschichten, wobei die äußere, konvexe Abdeckschicht die dickere Schicht ist, mit dem Vorteil, daß an der mechanischen äußeren Einwirkungen besonders ausgesetzten Oberfläche der Brustprothese durch die Abdeckschicht die höchste mechanische Festigkeit gegeben ist. So kann zum Beispiel die konkave Abdeckschicht etwa 1 bis 5 mm dick und die konvexe Abdeckschicht etwa 2 bis 8 mm dick sein.

Eine weitere Gewichtsreduzierung läßt sich noch dadurch erreichen, daß jede Abdeckschicht ein Maximum ihrer Dicke im Bereich der jeweiligen Wölbungsscheitel erhält und damit etwa sichelförmig ausgebildeten Querschnitt hat. Dies wirkt sich auch vorteilhaft auf die Formhaltung der Brustprothese aus, da größte mechanische Festigkeiten der stützenden Abdeckschicht in ihrem dickeren Bereich, also im Bereich der jeweiligen Wölbungsscheitel gegeben sind, und zwar dort, wo auch der Innenkern seine größte Dicke und damit Materialansammlung aufweist, die es zu stützen und zu schützen gilt.

Bei der erfindungsgemäß hergestellten Brustprothese befindet sich an den Grenzflächen zwischen Innenkern und Abdeckschichten sowie an den freien Oberflächen der Abdeckschichten jeweils wenigstens eine Kunststoffolie. Jede Folie besteht aus einem thermoplastischen Polyurethanpolymer und ist etwa 40 µ - 80 µ dick.

Die Kunststoffolien werden am Rand des schalenförmigen Prothesenkörpers miteinander verschweißt. Zwischen den Kunststoffolien werden somit jeweils der Innenkern und jeweils die Abdeckschichten eingeschlossen.

Die Herstellung der erfindungsgemäßen Brustprothese ist mit Vorteil einfach. Es werden mehrere, vorzugsweise vier Folien aufeinander gelegt und am Rand miteinander verschweißt, so daß sich zwischen den einzelnen Lagen entsprechende Foliensäcke gebildet haben. Die Schweißung kann in bestimmten Bereichen unterbrochen werden, so daß Einfüllöffnungen gebildet sind. Die noch leeren, aufeinanderliegenden Folien werden nunmehr gleichmäßig in einer Ebene auf einem Spannbrett oder einer Platte so aufgespannt, daß die Folien durch eine Dehnung von zum Beispiel 1 - 4 % elastisch vorgespannt sind. Danach können vorbestimmte Kammern, insbesondere die beiden äußeren Kammern mit einem Standard-Brustprothesen-Silikon-Gel als erstes Material gefüllt werden, wobei die dem Spannbrett zugekehrte Außenkammer etwa 20 - 70 %, vorzugsweise 50 % weniger Füllung erhält als die dem Spannbrett abgekehrte Außenkammer. Die zwischen den beiden jeweils innen liegenden Folien gebildete mittlere Innenkammer bleibt zunächst ungefüllt. Die natürliche Vorspannung sorgt dafür, daß sich das Silikon des ersten Materials gleichmäßig in den Außenkammern zwischen den Folien verteilt und an den Rändern gleichmäßig ausläuft. Durch Schrägstellen der Spannplatte läßt sich die Verteilung des Silikons noch beeinflussen. Es können im Spannbrett auch definierte Ausmuldungen vorhanden sein, welche die Verteilung des zweiten Materials ebenfalls beeinflussen können. Das Spannbrett mit dem in die Außenkammern eingefüllten ersten Material wird nunmehr in einen Ofen gesetzt und bei einer Temperatur ausvulkanisiert, bei der die Folien nicht verschweißen.

Nach dem ersten Ausvulkanisieren des ersten Materials wird das gesamte Folienpaket in eine Form bzw. ein entsprechendes Werkzeug eingespannt. Die mittlere Innenkammer zwischen den beiden inneren Folien des Folienpakets kann nunmehr innerhalb der geschlossenen Form, bzw. des geschlossenen Werkzeugs, mit einer als zweites Material vorgesehenen Mischung gefüllt werden. Die Mischung für das zweite Material besteht aus einem Zwei-Komponenten-Silikon-Gel, dem Leichtfüllstoffe, vorzugsweise Mikro-Glaskugeln, zugegeben worden sind. Es ist auch möglich, als Leichtfüllstoffe organische Füllstoffe, wie z.B. Kunststoff-Hohlkugeln zu verwenden.

Bei 140 °C bis 170°C, vorzugsweise 150°C, wird die gesamte Prothese nunmehr innerhalb von 1 bis 2 Stunden, vorzugsweise 75 Minuten, zum Beispiel in einem Umluftofen ausvulkanisiert. Dabei werden die insbesondere im Silikon zwischen erstem Material und zweitem Material eingebetteten Folien mit Vorteil mitverformt.

Nach dem Abkühlen wird die erfindungsgemäße Brustprothese aus dem Werkzeug entnommen und der überstehende Folienrand abgestanzt. Dabei wird als erfindungswesentlich angesehen, daß die Silikonschichten der äußeren Kammern vor dem Herstellen des Innenkerns bereits teilweise oder ganz ausvulkanisiert sind. Die Erfindung ermöglicht, Silikonteile mit elastischen Deckschichten zu versehen, ohne daß ein beträchtlicher Werkzeugaufwand erforderlich ist.

Ein Ausführungsbeispiel einer erfindungsgemäßen Brustprothese ist in der Zeichnung dargestellt. Es zeigen:
Fig. 1 eine Seitenansicht der Brustprothese im Schnitt und
Fig. 2 die Draufsicht der Brustprothese gemäß Fig. 1.

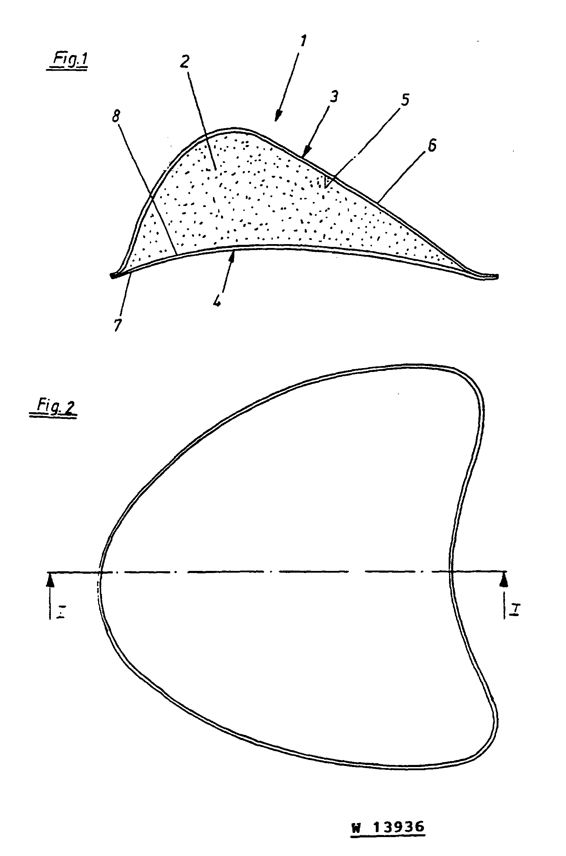

In Fig. 1 ist eine Seitenansicht einer Ausführungsform einer Brustprothese im Schnitt dargestellt. Die Brustprothese besteht aus einem Prothesenkörper 1, der etwa schalenförmig geformt ist. Der Prothesenkörper ist gebildet aus einem Innenkern 2, der an seiner konvexen Oberfläche mit einer Abdeckschicht 3 versehen ist und an seiner konkav gewölbten Oberfläche eine Abdeckschicht 4 hat.

Der Innenkern besteht aus einem zweiten Material, und zwar einem Silikon, dem 10 bis 25 %, vorzugsweise 16 % Leichtfüllstoffe zugesetzt sind, um das Gewicht des Innenkerns und damit der gesamten Prothese zu reduzieren. Für die Abdeckschichten 3 und 4 wird ein erstes Material verwendet, und zwar ein übliches Standard-Silikon.

Jede Abdeckschicht aus Standard-Silikon ist eingebettet zwischen zwei Folien 5,6;7,8 aus thermoplastischen Polyurethanelastomer mit einer Dicke von etwa 40 µ - 80 µ. Die Folien im Bereich der konvexen Oberfläche des Prothesenkörpers 1 sind mit 5 und 6 bezeichnet. Die Folien an der konkaven Oberfläche mit 7 und 8.

## Patentansprüche

1. Verfahren zur Herstellung einer Brustprothese mit einem etwa schalenförmigen Prothesenkörper, der gebildet wird durch Aufeinanderlegen von wenigstens zwei Kunststoffolien, die durch Randverbindung zu einem beutelartigen Behältnis mit mindestens einer Kammer ausgebildet werden und jede Kammer mit weichelastischem Material gefüllt wird, das nach Einlegen in ein Formwerkzeug einer Vulkanisierung unterzogen wird,
**dadurch gekennzeichnet,**
**daß** zunächst wenigstens eine Außenkammer mit einem ersten Material gefüllt wird, daß das in wenigstens einer der Außenkammern befindliche Material außerhalb des Formwerkzeuges zumindest teilweise ausvulkanisiert wird, daß anschließend wenigstens eine Innenkammer mit einem zweiten Material gefüllt wird und daß danach der so vorbereitete Prothesenkörper (1) im Formwerkzeug ausvulkanisiert wird.

2. Verfahren nach Anspruch 1, **dadurch gekennzeichnet, daß** vier Folien (5,6;7,8) aufeinander gelegt und am Rand miteinander so verschweißt werden, daß drei Kammern gebildet werden, daß die noch leeren aufeinanderliegenden Folien gleichmäßig in einer Ebene auf eine Platte aufgespannt werden und daß danach die beiden jeweils äußeren Außenkammern mit dem ersten Material gefüllt werden.

3. Verfahren nach Anspruch 2, **dadurch gekennzeichnet, daß** die der Platte zugekehrte Außenkammer mit etwa 20 - 70 % weniger erstem Material als die der Platte abgekehrte Außenkammer gefüllt wird.

4. Verfahren nach Anspruch 2, **dadurch gekennzeichnet, daß** die der Platte zugekehrte Außenkammer mit etwa 50 % weniger erstem Material als die der Platte abgekehrte Außenkammer gefüllt wird.

5. Verfahren nach einem der Ansprüche 2 - 4, **dadurch gekennzeichnet, daß** die Verteilung des ersten Materials in den Außenkammern durch Schrägstellen der Platte beeinflußt wird.

6. Verfahren nach einem der Ansprüche 2 - 5, **dadurch gekennzeichnet, daß** die Platte nach Füllung der Außenkammern in einen Ofen gesetzt und das erste Material in den Außenkammern bei einer Temperatur vulkanisiert wird, bei der die Folien nicht verschweißen.

7. Verfahren nach einem der Ansprüche 1 - 6, **dadurch gekennzeichnet, daß** das gesamte Folienpaket mit den zumindest teilweise vulkanisierten Außenkammern von der Platte abgenommen und in ein Formwerkzeug eingespannt wird, daß das Formwerkzeug geschlossen wird, daß eine zwischen einander benachbarten Innenfolien (5,8) befindliche Innenkammer mit dem zweiten Material gefüllt wird und daß das Formwerkzeug in einen Ofen gesetzt und die gesamte Prothese ausvulkanisiert wird.

8. Verfahren nach einem der vorhergehenden Ansprüche, **dadurch gekennzeichnet, daß** als zweites Material für den Innenkern (2) eine mit Leichtfüllstoff versetzte Silikon-Masse mit einer Dichte von 0,3 g/cm³ bis 0,8 g/cm³, vorzugsweise 0,64 g/cm³ verwendet wird.

9. Verfahren nach Anspruch 8, **dadurch gekennzeichnet, daß** als Leichtfüllstoffe Mikro-Glaskugeln verwendet werden.

10. Verfahren nach Anspruch 8, **dadurch gekennzeichnet, daß** als Leichtfüllstoffe organische Füllstoffe verwendet werden.

11. Verfahren nach Anspruch 1, **dadurch gekennzeichnet, daß** als zweites Material ein Polyurethanschaum verwendet wird.

12. Verfahren nach Anspruch 1, **dadurch gekennzeichnet, daß** als zweites Material ein Silikon-Schaum verwendet wird.

13. Verfahren nach einem der Ansprüche 1 bis 11, **dadurch gekennzeichnet, daß** als erstes Material eine übliche additionsvernetzte Zwei-Komponenten-Silikon-Kautschuk-Masse verwendet wird.

## Claims

1. A method for producing a breast prosthesis with an approximately shell-like prosthesis body formed by placing one on top of the other at least two plastic films which are constructed by joining the edges into a bag-like container with at least one chamber and each chamber is filled with soft-elastic material which is subjected to vulcanisation after insertion into a mould,
**characterised in that**
at least one outer chamber is filled with a first material, that the material located in at least one of the outer chambers is at least partly vulcanised out outside the mould, that at least one inner chamber is then filled with a second material and that the prosthesis body (1) thus prepared is then vulcanised out in the mould.

2. The method according to claim 1, **characterised in that** four films (5, 6; 7, 8) are placed one on top of the other and welded together at the edge so that three chambers are formed, that the still empty films placed one on top of the other are then uniformly clamped on a plate in one plane and the two respectively outer chambers are then filled with the first material.

3. The method according to claim 2, **characterised in that** the outer chamber facing the plate is filled with approximately 20 - 70 % less first material than the outer chamber facing away from the plate.

4. The method according to claim 2, **characterised in that** the outer chamber facing the plate is filled with approximately 50 % less first material than the outer chamber facing away from the plate.

5. The method according to any one of claims 2 - 4, **characterised in that** the distribution of the first material in the outer chambers is influenced by tilting the plate.

6. The method according to any one of claims 2 - 5, **characterised in that** after filling the outer chambers, the plate is placed in a furnace and the first material in the outer chambers is vulcanised at a temperature at which the films do not weld together.

7. The method according to any one of claims 1 - 6, **characterised in that** the total film package with the at least partly vulcanised outer chambers is removed from the plate and clamped in a mould, that the mould is closed, that an inner chamber located between adjacent inner films (5, 8) is filled with the second material and that the mould is placed in a furnace and the entire prosthesis is vulcanised out.

8. The method according to any one of the preceding claims, **characterised in that** a silicone mass mixed with a light filler having a density of 0.3 g/cm³ to 0.8 g/cm³, preferably 0.64 g/cm³ is used as the second material for the inner core (2).

9. The method according to claim 8, **characterised in that** micro-glass balls are used as light fillers.

10. The method according to claim 8, **characterised in that** organic fillers are used as light fillers.

11. The method according to claim 1, **characterised in that** a polyurethane foam is used as the second material.

12. The method according to claim 1, **characterised in that** a silicone foam is used as the second material.

13. The method according to any one of claims 1 to 11, **characterised in that** a common addition-cross-linked two-component silicone-rubber mass is used as the first material.

## Revendications

1. Procédé de production d'une prothèse mammaire présentant un corps de prothèse en forme de coque, formé par superposition d'au moins deux feuilles de plastique qui par réunion des bords, forment un conteneur de type sac présentant au moins une chambre, chaque chambre étant remplie d'un matériau élastique mou, qui est soumis à une vulcanisation après placement dans un outil de formage, **caractérisé en ce que**, au moins une chambre externe est tout d'abord remplie avec un premier matériau, **en ce que** le matériau se trouvant dans au moins l'une des chambres extérieures en dehors de l'outil de formage est vulcanisé au moins en partie, **en ce qu'**ensuite au moins une chambre intérieure est remplie avec un deuxième matériau et qu'ensuite, le corps de prothèse (1) ainsi formé est vulcanisé dans l'outil de formage.

2. Procédé selon la revendication 1, **caractérisé en ce que** quatre feuilles (5, 6 ; 7, 8) sont superposées et soudées les unes aux autres sur le bord de telle sorte que trois chambres soient formées, **en ce que** les feuilles superposées encore vides sont tendues uniformément en un plan sur une plaque et qu'ensuite, les deux chambres extérieures respectives sont remplies avec le premier matériau.

3. Procédé selon la revendication 2, **caractérisé en ce que** la chambre extérieure retournée vers la plaque est remplie avec environ 20 à 70 % de moins du premier matériau par rapport à la chambre extérieure détournée de la plaque.

4. Procédé selon la revendication 2, **caractérisé en ce que** la chambre retournée vers la plaque est remplie d'environ 50 % de moins du premier matériau par rapport à la chambre extérieure détournée de la plaque.

5. Procédé selon l'une quelconque des revendications 2 à 4, **caractérisé en ce que** la répartition du premier matériau dans la chambre extérieure est influencée par la position oblique de la plaque.

6. Procédé selon l'une quelconque des revendications 2 à 5, **caractérisé en ce que** la plaque est placée dans un four après le remplissage de la chambre extérieure et que le premier matériau dans la chambre extérieure est vulcanisé à une température à laquelle les feuilles ne se soudent pas.

7. Procédé selon l'une quelconque des revendications 1 à 6, **caractérisé en ce que** l'ensemble des feuilles avec la chambre extérieure vulcanisée au moins en partie est prélevé de la plaque et est tendu dans un outil de formage, **en ce que** l'outil de formage est fermé, qu'une chambre intérieure se trouvant entre les feuilles intérieures voisines l'une de l'autre (5, 8) est remplie avec le deuxième matériau et que l'outil de formage est placé dans un four, la prothèse complète étant vulcanisée.

8. Procédé selon l'une quelconque des revendications précédentes, **caractérisé en ce que**, comme deuxième matériau pour la chambre intérieure (2), on utilise une masse de silicone mélangée avec un matériau de remplissage léger présentant une densité comprise entre 0,3 g/cm³ et 0,8 g/cm³, de préférence de 0,64 g/cm³.

9. Procédé selon la revendication 8, **caractérisé en ce que** l'on utilise comme matériau de remplissage léger, des microbilles de verre.

10. Procédé selon la revendication 8, **caractérisé en ce que** l'on utilise comme matériau de remplissage léger, une substance de remplissage organique.

11. Procédé selon la revendication 1, **caractérisé en ce que** l'on utilise comme deuxième matériau, une mousse de polyuréthane.

12. Procédé selon la revendication 1, **caractérisé en ce que** l'on utilise comme deuxième matériau, une mousse de silicone.

13. Procédé selon l'une quelconque des revendications 1 à 11, **caractérisé en ce que** l'on utilise comme premier matériau, une masse à deux composants silicone-caoutchouc réticulée par addition de manière classique.
